Europäisches Patentamt

European Patent Office

Office européen des brevets

(11) Numéro de publication: **0 219 372**
**A1**

# (12) DEMANDE DE BREVET EUROPEEN

(21) Numéro de dépôt: **86401911.2**

(22) Date de dépôt: **29.08.86**

(51) Int. Cl.⁴: **C 12 N 5/00**
**A 23 C 9/142**

(30) Priorité: **29.08.85 FR 8512907**

(43) Date de publication de la demande:
**22.04.87 Bulletin 87/17**

(84) Etats contractants désignés:
**AT BE CH DE FR GB IT LI LU NL SE**

(71) Demandeur: **UNIVERSITE DE NANCY**
**24, rue Lionnois, B.P. 3137**
**F-54013 Nancy Cédex (FR)**

(72) Inventeur: **Linden, Guy**
**21, rue de Brest**
**F-54180 Heillecourt (FR)**

**Nabet, Pierre**
**7, rue Catherine de bourbon**
**F-54140 Jarville (FR)**

**Sarem, Farzin**
**3, rue de Verviers Appt 73 Les Bisets**
**F-54500 Vandoeuvre Les Nancy (FR)**

**Le Deaut, Jean-Yves**
**21, rue Messier**
**F-54000 Nancy (FR)**

(74) Mandataire: **Peaucelle, Chantal et al**
**S.C. Ernest Gutmann - Yves Plasseraud 67, boulevard Haussmann**
**F-75008 Paris (FR)**

(54) **Fractions de lait, procédé d'obtention de ces fractions et milieux de culture cellulaires renfermant ces fractions.**

(57) L'invention concerne des fractions de lait formées de constituants de poids moléculaire supérieur à 27.000 ou lorsqu'il s'agit de lait thermisé de poids moléculaire supérieur à 10.000, dépourvus de lipides insolubles du type de ceux éliminables par ultracentrifugation. Ces fractions sont utilisables pour supplémenter des milieux de cultures cellulaires en remplacement du sérum, pour congeler et conserver ces cellules.

EP 0 219 372 A1

## Description

Fractions de lait, procédé d'obtention de ces fractions et milieux de culture cellulaires renfermant ces fractions.

L'invention a pour objet des fractions de lait utilisables notamment comme milieux de cultures cellulaires, leur procédé d'obtention et les milieux de cultures les renfermant.

On sait que de nombreux milieux de cultures cellulaires sont supplémentés en sérum, mettant ainsi à profit les propriétés d'induction de croissance cellulaire de divers facteurs qu'ils renferment.

Compte tenu cependant du coût élevé du sérum, divers substituts ont été envisagés, notamment un autre fluide corporel, à savoir le lait.

Dans Proc. Nat. Acad. Sci. USA N° 10, p. 5057-5061, Octobre 1978, Klagsbrun rapporte des résultats de travaux effectués avec du lait humain, qui ont montré un effet de stimulation de la synthèse d'ADN et de la division cellulaire de fibroblastes BALB/c 3T3. Par gel filtration, un facteur mitogène de poids moléculaire (PM) de 14 000 à 18 000 daltons a été isolé.

Dans des articles postérieurs, notamment dans Journal of Cell Biology, vol. 88, Février 1981, p. 294-300, et dans Journal of Cellular Physiology 109 : 223-234 (1981), Steimer et coll. soulignent cependant que le lait, et dans une moindre mesure le colostrum, sont déficients en facteurs d'attachement présents dans le sérum tels que la fibronectine. Il s'ensuit que, dans le cas de culture de cellules non transformées dans des milieux de cultures supplémentés en lait, il est néccessiare de prétraiter les plaques de culture avec de la fibronectine pour permettre l'attachement des cellules. Les résultats rapportés montrent toutefois que, même dans ces conditions, les lignées cellulaires normales de fibroblastes ne prolifèrent pas de manière appréciable.

Les travaux des inventeurs dans ce domaine les ont conduits à constater qu'il est possible de disposer d'un substitut de sérum économique et de grande efficacité en fractionnant le lait dans des conditions déterminées.

L'invention a donc pour but de fournir des fractions de lait utilisables en remplacement du sérum dans les milieux de culture cellulaires.

Elle vise également à fournir un procédé d'obtention, de mise en oeuvre aisée, de ces fractions.

Elle vise en outre à fournir des milieux de cultures cellulaires artificiels, économiques, de grande efficacité.

Le terme "lait", tel qu'utilisé dans la description et les revendications, désigne aussi bien le lait de vache que le lait d'autres mammifères tels qu chèvre, brebis, buflonne, jument, yack ou chamelle.

Les fractions de lait de l'invention sont caractérisées en ce qu'il s'agit de fractions de lait thermisé, c'est-à-dire de lait ayant subi un préchauffage à une température inférieure à 60° C environ pendant au moins 5 à 10 mn, qu'elles sont essentiellement dépourvues de lipides insolubles du type de ceux éliminables par une opération telle que l'ultra centrifugation et des inhibiteurs de poids moléculaire inférieur à environ 1000, et qu'elles sont essentiellement formées de constituants de poids moléculaire d'au moins environ 10000.

Ces fractions contiennent les protéines solubles du lait, en particulier des glycoprotéines, les immunoglobulines, principalement des Ig G, A et M, de la sérumalbumine, des protéases et des peptones.

Selon un autre aspect, les fractions de lait de l'invention sont caractérisées en ce qu'elles sont formées de constituants ayant un PM d'au moins 27.000 et qu'elles sont essentiellement dépourvues de lipides insolubles du type de ceux éliminables par ultracentrifugation ou analogue et des inhibiteurs de poids moléculaire inférieur à environ 1000.

D'une manière surprenante, l'addition à des milieux de culture conventionnels, qui sont donc dépourvus du facteur mitogène évoqué ci-dessus, de fractions du type ci-dessus, conduit cependant à une stimulation de la croissance des cellules. La prolifération cellulaire observée est supérieure à celle obtenue avec des fractions formées de constituants de PM inférieur à 27.000 ou dans le cas de lait thermisé de PM inférieur à 10.000 ou encore avec du lait entier non fractionné, ce que ne laissaient prévoir les indications figurant dans le brevet US 4.440.860 du 2 Mars 1981 au nom de Klagsbrun. En effet, selon ce brevet, les protéines ayant un poids moléculaire supérieur au facteur mitogène ci-dessus sont considérées comme des protéines inactives. Or, les travaux des inventeurs ayant abouti à la mise au point de cette invention montrent au contraire l'efficacité des fractions de poids moléculaire éléve, plus spécialement supérieur à 50 000.

Ainsi, des fractions préférées sont formées de constituants de PM supérieur à 50 000 environ.

D'autres fractions préférées présentent des PM de 27 000 à 50 000 environ. Ces fractions sont soit entières renfermant en mélange les constituants dont les PM sont supérieurs à 50 000, ou bien s'étagent de 27 000 à 50 000, soit fractionnées dans cet intervalle de PM.

D'autres fractions préférées sont encore obtenues à partir de lait thermisé et elles présentent un PM d'au moins 10.000 environ.

Ces fractions sont avantageusement du type de celles obtenues par ultracentrifugation du lait thermisé à 50.000 g au plus, de préférence entre 30 et 40.000 g environ, suivie d'au moins une ultracentrifugation dans des conditions analogues de la phase intermédiaire limpide recueillie, puis d'au moins une étape d'ultrafiltration sur membranes afin d'éliminer les molécules de PM inférieur à environ 10.000.

D'une manière avantageuse, ces différentes fractions sont débarrassées de molécules à effet inhibiteur de PM inférieur à 1000.

Les fractions de l'invention sont constituées par des fractions de lait frais, de lait pasteurisé, de lait thermisé, sous forme liquide concentrée ou lyophilisée.

Selon un autre aspect, les fractions de l'invention sont du type de celles obtenues après ultracentrifugation pour éliminer des lipides insolubles. L'ultracentrifugation est réalisée selon les méthodes classiques et notamment à une température inférieure à l'ambiante, en particulier inférieure à 10°C, notamment de l'ordre de 4°C, pendant une durée de l'ordre de 60 minutes notamment à environ 100 000 g (ce qui correspond à 20 000 tours environ par minute), récupération de la phase limpide intermédiaire et ultrafiltration de cette phase intermédiaire sur une membrane ayant un seuil de coupure approprié dans l'intervalle de 50 000 à 27 000 daltons environ ou pour les fractions de lait thermisé, un seuil de coupure de 10.000 daltons environ.

Conformément au procédé de l'invention, on soumet une phase limpide de lait, renfermant les protéines solubles du lait, telle qu'obtenue par ultracentrifugation du lait et séparation à partir des différentes phases obtenues, à au moins une ultrafiltration avec une membrane à seuil de coupure de 50.000 à 27.000 daltons environ ou pour le lait thermisé à seuil de coupure de 10.000 daltons.

On notera que l'utilisation de la phase limpide ci-dessus permet une stérilisation plus aisée par filtration.

De préférence, on sépare tout d'abord à l'aide d'une membrane à seuil de coupure de 50 000 daltons la fraction de lait formée de constituants de PM supérieur à 50 000, puis on soumet la fraction restante à une ultrafiltration avec une membrane ayant un seuil de coupure approprié pour l'obtention d'une fraction formée de constituants présentant des PM dans l'intervalle de PM désiré jusqu'à 50 000 daltons.

La fraction obtenue après ultrafiltration est concentrée. Avant d'être stérilisée, la fraction est avantageusement préfiltrée par exemple sur un filtre de dimensions de pores de 0,45 µ environ.

La fraction peut être conservée par congélation par exemple à -80°C environ jusqu'à son utilisation. Au moment de l'utilisation, celle-ci est diluée et avantageusement filtrée sur filtre d'environ 0,22 µ.

La phase de lait limpide mise en oeuvre dans l'étape d'ultrafiltration est obtenue avantageusement en soumettant le lait à au moins une opération d'ultracentrifugation. L'ultracentrifugation est réalisée plus spécialement à 27 000 t/mn environ durant 60 mn environ, à une température inférieure à l'ambiante, avantageusement inférieure à 10°C, notamment de l'ordre de 4°C.

Le lait mis en oeuvre est constitué plus spécialement par du lait frais. Il peut s'agir également de lait pasteurisé ou de lait thermisé. Comme l'ont montré les résultats des essais effectués par la demanderesse, les fractions de lait ainsi obtenues exercent un effet de stimulation de la synthèse d'ADN et de la division cellulaire plus important que celui obtenu avec du lait entier ou des fractions de PM inférieur à 10.000.

L'addition des fractions de l'invention à des milieux de culture conventionnels permet d'élaborer des milieux de culture artificiels, économiques par rapport à ceux supplémentés en sérum.

L'invention vise donc des milieux de cultures cellulaires caractérisés en ce qu'ils comprennent un milieu de base conventionnel supplémenté à l'aide d'une fraction selon l'invention en une quantité suffisante pour permettre de réduire ou de supprimer l'utilisation de sérum tout en exerçant un effet activateur sur le prolifération cellulaire. Selon le type et les besoins des cellules, le milieu de base est supplémenté, de manière classique, en sources de carbone et d'énergie différentes. En outre, l'addition d'antibiotiques permet d'éviter les contaminations.

D'une manière générale, on sait que tous les facteurs de croissance exercent une action biphasique sur la croissance cellulaire. A faible concentration, ces facteurs activent la croissance cellulaire jusqu'à une concentration optimale. Au-delà de cette concentration et à forte concentration, ils inhibent la croissance.

La concentration en fractions de lait des milieux de cultures de l'invention sera aisément déterminée par l'homme de l'art compte tenu du type des cellules à cultiver.

Des milieux de cultures préférés, particulièrement appropriés notamment à la croissance d'hybridomes et de fibroblastes, comprennent un milieu de base supplémenté à l'aide de fractions de lait formées de constituants de PM supérieur à environ 50 000, ou de fractions de lait thermisé formées de constituants de PM supérieur à 10.000 environ.

Des concentrations d'environ 6 à 10 % (vol/vol) permettent d'obtenir d'une manière générale une activité maximale.

Ainsi, la stimulation de la synthèse d'ADN est supérieure à 50 % avec de telles fractions par rapport à celle obtenue avec la phase limpide non fractionnée. De telles fractions présentent une activité d'au moins 86 % par rapport à un sérum de veau foetal (SVF) alors que des fractions formées de constituants de PM inférieur à 27 000, en particulier inférieur à 10 000 et même à 1000 entraînent seulement une stimulation inférieure à 15 % par rapport à SVF.

L'addition de 1 à 10 % (vol/vol) de sérum, de préférence de 3 à 5%, peut être avantageuse pour obtenir une activité maximale du milieu de culture et/ou pour des cultures à long terme, ce qui constitue une réduction appréciable de la quantité de sérum à utiliser par une culture cellulaire donnée.

Il apparaît ainsi qu'une fraction par exemple formée de constituants de PM supérieur à 50 000 daltons, à une concentration de 10 %, additionnée de 1 % de SVF, présente une activité équivalente à celle obtenue avec un milieu contenant 10 % de SVF.

L'invention fournit donc des moyens pour réduire le coût des milieux de culture en diminuant la quantité de sérum à utiliser.

Ces milieux sont mis en oeuvre avec avantage pour la culture de cellules d'eucaryotes, entre autres, hybridomes, fibroblastes, hépatocytes, lymphoïdes, cellules épithéliales.

D'une manière avantageuse, les cellules d'eucaryotes peuvent être conservées par congélation dans un milieu de culture tel que défini ci-dessus. Après décongélatation, les cellules sont reprises dans un milieu de culture tel qu'utilisé au départ.

D'autres caractéristiques et avantages de l'invention sont donnés dans la description des exemples qui suivent et apparaîtront également en se référant aux figures :

- la figure 1 représentant les courbes de variation du nombre de cellules d'hybridomes par ml en fonction du temps,

- la figure 2 représentant les courbes de variation du nombre de cellules de fibroblastes ayant incorporé la thymidine tritiée en fonction de la concentration de la fraction testée.

- la figure 3 représentant les courbes de variation du nombre de cellules d'hybridomes ayant incorporé la thymidine tritiée, en fonction de la nature de la fraction de lait testée.

- la figure 4 représentant les courbes de variation de la concentration en cellules d'hybridomes par ml, en fonction du temps, lorsque le lait de départ est préchauffé dans les conditions précisées.

- et la figure 5 représentant les courbes de variation de la division des cellules d'hybridomes après congélation suivie d'une décongélation, en fonction du temps.

Exemple 1 : Fractionnement de lait frais de vache.

En opérant dans des conditions aseptiques, on recueille du lait frais 60 heures environ après le début de la lactation dans des flacons stériles fermés ensuite hermétiquement. Le lait recueilli est soumis à deux opérations d'ultracentrifugation, puis à une ultrafiltration.

a) Ultracentrifugation.

Le lait frais est soumis à une opération d'ultracentrifugation (ultracentrifugeuse Beckmann Model L2-65B rotor SW27), à deux reprises, à 100 000 g (20 000 tours/minute) durant 60 minutes à 4°C.

Après l'ultracentrifugation, trois phases apparaissent, à savoir :

- une phase supérieure ou phase lipidique prélevée avec précaution afin de ne pas troubler l'ultracentrifugat,
- une phase intermédiaire limpide, prélevée et conservée à 4°C avant l'étape d'ultrafiltration ; cette phase est appelée ci-après LFLac,
- une phase inférieure ou caséinique.

b) Ultrafiltration.

On filtre 170 ml de LFLac dans une cellule Amicon (modèle 402 75 PSI MAX) sous agitation constante, sous une pression de 4 bars.

Les membranes utilisées sont des membranes Millipore à seuil de coupure de 50 000 daltons et le cas échéant pour effectuer des essais comparatifs de 27 000 et 1000 daltons.

L'ultrafiltration sur membrane 50 000 est arrêtée lorsque les volumes du retentat et de l'ultrafiltrat sont respectivement de 1/7 et 6/7 du volume initial. Le retentat contenant les fractions renfermant les molécules de PM supérieur à 50 000 (désignées ci-après LFLac 50)(concentration x 7) est congelé à -80°C en aliquotes de 5 ml.

l'ultrafiltrat est soumis le cas échéant à une deuxième ultrafiltration sur une membrane à seuil de coupure de 27 000 daltons. Le volume initial est de 160 ml. 30 ml de retentat sont recueillis et lavés ; ils contiennent les fractions renfermant les molécules comprises entre 27 000 et 50 000 daltons (concentration x 4). Ces fractions sont appelées ci-après LFLac 50-27.

Aux fins d'utilisation dans des essais comparatifs, l'ultrafiltrat à PM inférieur à 27 000 daltons est soumis à une troisième ultrafiltration sur une membrane de 1000 daltons. Le retentat et l'ultrafiltrat recueillis contiennent respectivement des fractions renfermant des molécules à PM compris entre 1000 et 20 000 daltons, fractions LFLac 10-1, et des fractions LFLac formées de molécules à PM inférieur à 1000 daltons. Les retentats et ultrafiltrats sont conservés congelés à -80°C en aliquotes de 5 ml.

Exemple 2 : Fractionnement de lait de vache pasteurisé.

On prépare les deux fractions suivantes :

- une fraction LPLacE, en opérant comme pour le lait frais, puis en stérilisant sur membrane 0,45 μ ; cette fraction est constituée par la phase intermédiaire limpide obtenue après ultracentrifugation ;
- la fraction LPLac 50 par ultracentrifugation puis passage sur membrane à seuil de coupure 50 000 et récupération du retentat formé de produits de PM supérieur à 50 000.

Exemple 3 : Utilisation de fractions de lait frais selon l'invention comme milieu de culture d'hybridomes.

On indique ci-après a) la nature des hybridomes utilisés dans les essais dont les résultats sont rapportés dans cet exemple, b) la nature du milieu de culture et c) les méthodes de mesure de la croissance cellulaires.

a) Hybridomes

Les hybridomes sont issus d'une hybridation entre les cellules de myélomes Ag 8 de souris et de plasmocytes secrétant des anti-β lactoglobulines bovines.

Les cellules d'hybridomes mises en oeuvre sont en phase de croissance exponentielle dans un milieu avec 10 % de SVF. Ces cellules sont réparties sur une microplaque de 24 puits à raison de $10^5$ cellules/puits dans 100 μl de milieu supplémenté et renferment les différents produits à tester.

b) Milieu de culture.

Le milieu de base utilisé est le milieu synthétique RPMI 1640 (commercialisé par Boehringer). Ce milieu renferme :

- des acides aminés (excepté la L-glutamine en raison de son instabilité) ;
- du rouge de phénol (indicateur de pH) ;
- un tampon bicarbonate ($NaHCO_3$) qui sert à maintenir le $CO_2$ présent dans le milieu en équilibre ainsi qu'au métabolisme de certaines cellules.

Ce milieu est supplémenté afin de répondre à la composition suivante :

RPMI 1640 500 ml
L-glutamine 200 mM 5 ml
Glucose 30 % 5 ml
Pyruvate 1 % 6 ml
β-mercaptoéthanol 5 μl/ml 1 ml
Pénicilline 100 μ/ml 400 μl
Streptomycine 100 μg/ml 200 μl

c) Mesure de la croissance cellulaire.

α) On détermine la quantité d'ADN synthétisé en fonction du temps avec différentes concentrations des fractions à tester en effectuant un comptage de radioactivité après incorporation de thymidine tritiée.

Après l'ajout des fractions à tester, les cultures sont mises à incuber pendant 24 heures à 37°C. Ces 24 heures représentent un cycle cellulaire.

Au bout de 24 heures, la thymidine tritiée (solution aqueuse de 20 Ci/mmole Amersham) est ajoutée à une concentration de 1 μCi/ml. 50 μl sont ajoutés aux cultures d'hybridomes. On laisse le milieu incuber pendant 18 à 20 heures à 37°C.

Après incorporation de la thymidine tritiée, les cellules sont récupérées pour être comptées (compteur : liquide scintillation spectrometer Packard Tri-card).

On effectue un comptage de la radioactivité (rayonnement β émis par le tritium) dans le compteur.

Aux fins d'analyse et de comparaison des résultats, une standardisation a été effectuée par rapport à un milieu de référence constitué par un milieu RPMI renfermant 10 % de SVF. On utilise SVF commercialisé par Boehringer Mannheim GmbH, filtré sur membrane 0,22 μm avant d'être ajouté au milieu.

Les essais effectués à différentes concentrations de LFLac 50 montrent que cette fraction stimule l'incorporation de thymidine tritiée à partir d'une concentration de 10 %.

β) La cinétique de croissance est réalisée par comptage des cellules en fonction du temps et de la concentration des produits à tester.

Deux modes opératoires sont rapportés, l'un comprenant un changement régulier de milieu toutes les 48 heures et le deuxième étant réalisé sans changement de milieu de culture jusqu'à épuisement.

La croissance est étudiée sur milieu RPMI supplémenté avec les milieux à tester.

Il s'agit de plusieurs mélanges : soit SVF 1 % seul, soit SVF 10 % seul, soit LFLac 50 10 % + SVF 1 %.

1) Sans changement de milieu.

$2.10^5$ cellules sont placées dans 8 ml de milieu de croissance dans une boîte de 25 cm² (Falcon).

Les cellules sont en phase exponentielle de croissance. Le comptage est réalisé toutes les 24 heures jusqu'à épuisement du milieu qui vire au jaune.

2) Avec changement de milieu.

Dans ce cas, on met en culture $6.10^5$ cellules dans 30 ml de milieu de croissance. Après 48 heures d'incubation, on retire 20 ml de milieu très doucement afin de ne pas mettre les cellules en suspension. On agite les 10 ml qui restent au fond de la boîte et on en prélève une goutte pour le comptage. On rajoute enfin 20 ml de milieu de croissance frais. On effectue la même opération toutes les 48 heures pendant 3 à 4 jours.

Dans le tableau 1 qui suit, on rapporte les valeurs moyennes du nombre de cellules par ml obtenues sur un nombre n de cupules. Le comptage est effectué au microscope à grossissement x 125.

## TABLEAU 1

| Heures<br>Fractions | 48 | 96 | 120 | 144 | 168 |
|---|---|---|---|---|---|
| LFLac 50 10 %<br>+ SVF 1 % | $40 \times 10^4$ | $20 \times 10^5$ | $26 \times 10^5$ | $42 \times 10^5$ | $18 \times 10^5$ |
| SVF 1 % | $24 \times 10^4$ | $92 \times 10^5$ | $92 \times 10^4$ | $76 \times 10^4$ | $52 \times 10^4$ |

Ces résultats montrent que LFLac 50 10 % en présence seulement de SVF 1 % entraîne une prolifération cellulaire importante, et ce, avantageusement, à long terme.

La comparaison des résultats obtenus avec SVF 1 % et SVF 10 % permet d'établir les rapports suivants :

$$\frac{\text{Prolifération maximale sur LFLac 50 10\% + SVF 1\%}}{\text{Prolifération maximale sur SVF 10\%}} = 0,39$$

$$\frac{\text{Prolifération maximale sur LFLac 50 10\% + SVF 1\%}}{\text{Prolifération maximale sur SVF 1\%}} = 29$$

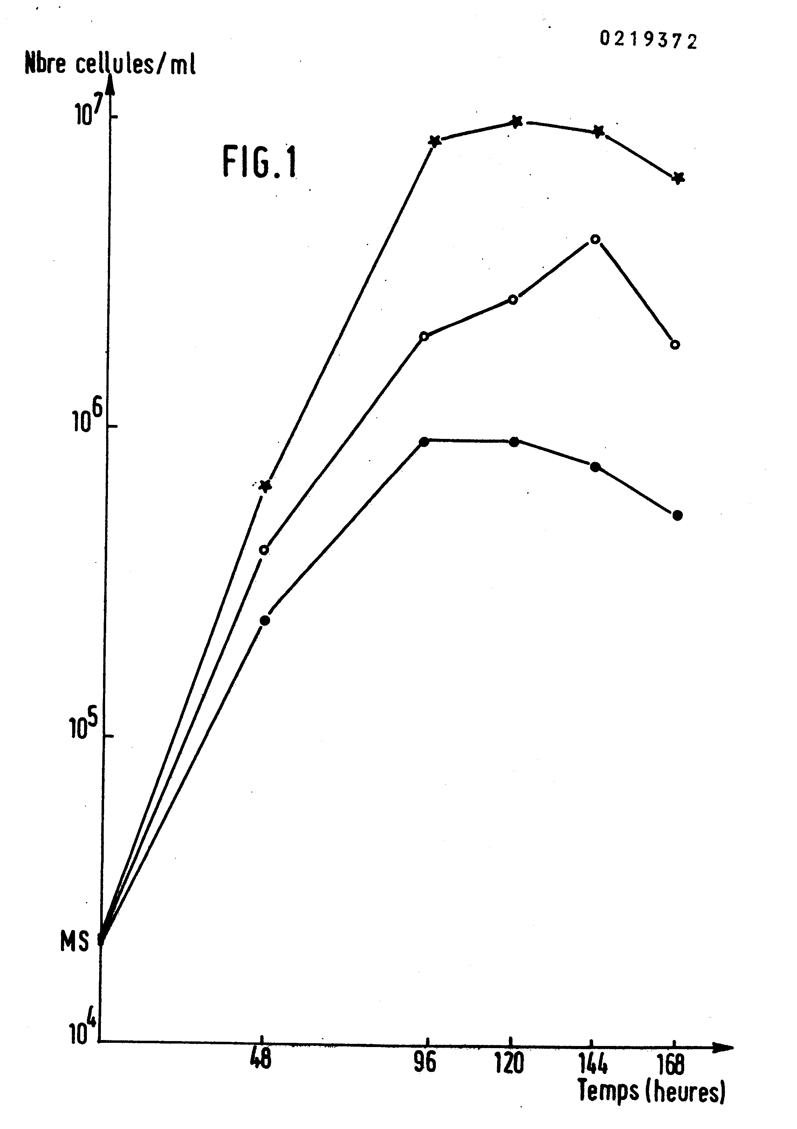

Sur la figure 1, on a également rapporté les courbes de variation en fonction du temps (en heures) du nombre de cellules d'hybridomes par ml dans le cas de la croissance sur SVF 10 % (courbe *), sur SVF 1 % + LFLac 50 10 % (courbe *) et sur SVF 1 %. L'examen de cette figure met en évidence l'ensemble des résultats considérés ci-dessus.

Exemple 4 : Utilisation de fractions de lait pasteurisé selon l'invention pour des cultures d'hybridomes à long terme.

On effectue les cultures dans un milieu RPMI 1640 contenant LPLac > 27 000 10 % + SVF 1 % changé régulièrement. La prolifération cellulaire est maintenue pendant plusieurs semaines.

Exemple 5 : Utilisation de fractions de lait frais selon l'invention comme milieux de culture de fibroblastes.

Les résultats rapportés ci-après concernent des cellules de fibroblastes obtenues à partir d'une biopsie de peau d'un enfant de 4 ans, entre le 7ème et le 9ème passage.

Le milieu de culture utilisé pour les fibroblastes est beaucoup moins riche et n'est supplémenté qu'avec de la L-glutamine et des antibiotiques (dans la même proportion que pour les hybridomes).

Les fibroblastes détachés et dissociés sont répartis dans les 24 puits des microplaques à raison de 3 à $4.10^4$ cellules/ml et à raison de 1 ml par puits. Ils sont laissés 24 heures dans le milieu RPMI 1640 supplémenté de 10 % de SVF pour fixation, puis lavés deux fois avec 2 ml de milieu RPMI et remis 48 heures dans le milieu sans sérum, ce qui les synchronise en phase Go/Gl. Toutes les expériences se font sur des cellules non confluentes.

On mesure l'incorporation de thymidine tritiée en opérant comme décrit pour les hybridomes dans l'exemple 4, le temps d'incubation avec les fractions testées étant de 48 heures.

Dans le tableau 2 suivant, on indique les activités moyennes standardisées (coups par 5 minutes de thymidine tritiée incorporée avec standardisation par rapport à un milieu de référence constitué par un milieu RPMI renfermant 10 % de SVF) à différentes concentrations de SVF et de LFLac 50.

## TABLEAU 2

| Concentrations | 1 % | 5 % | 10 % | 20 % | Milieu seul |
|---|---|---|---|---|---|
| Fractions | | | | | |
| SVF | 1 628 | 3 014 | 3 445 | 5 306 | 168 |
| LFLac 50 | 758 | 2 486 | 2 951 | 3 497 | 168 |

Les résultats standardisés permettent d'observer une incorporation de thymidine tritiée importante avec la fraction LFLac 50 ; cette incorporation augmente proportionnellement à la quantité de fraction ajoutée au milieu.

A une concentration de 10 % de LFLac 50, la stimulation de synthèse de DNA des cellules équivaut à 86 % de celle observée avec SVF 10 % (vol/vol).

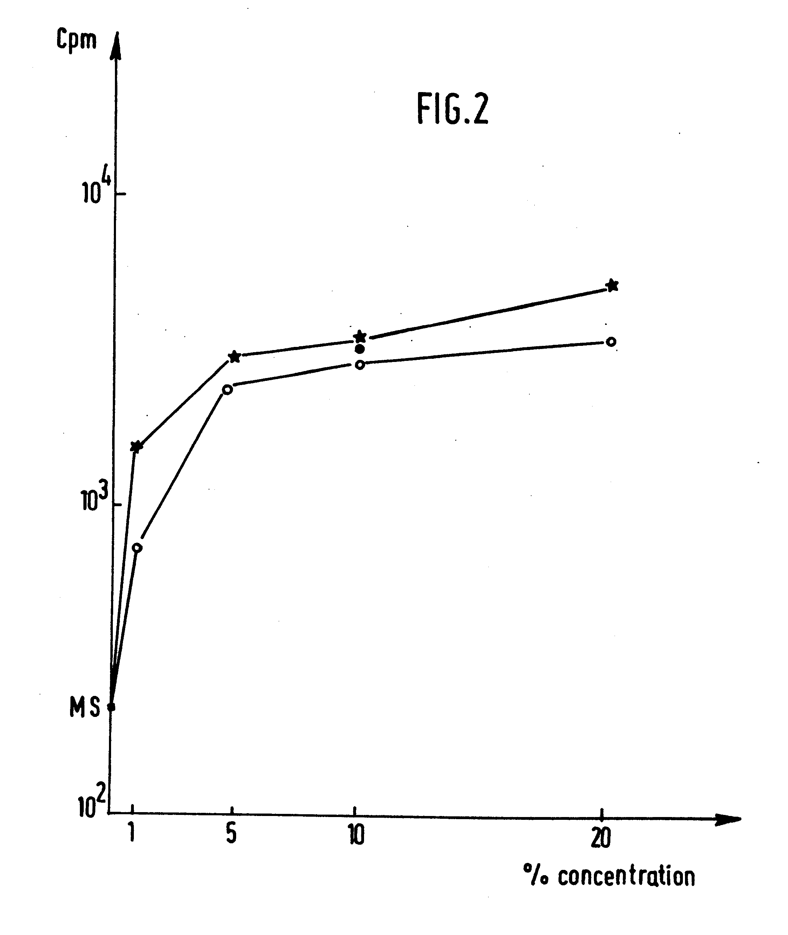

Ces résultats sont également illustrés par la figure 2 qui comprend les courbes de variation, en fonction de la concentration des fractions à tester, de l'activité moyenne standardisée de la culture cellulaire, la courbe * concernant la croissance sur SVF, la courbe o la croissance sur LFLac 50 et la courbe * la croissance sur SVF 1 % + LFLac 50 10 %.

Une série d'expériences similaires a été réalisée avec des fractions LFLac 50 10 % en présence de SVF 1 % et à titre de comparaison avec SVF 10 % et avec SVF 1 %.

On rapporte dans le tableau 3 suivant le nombre de cellules/ml (moyenne de deux essais) à différents temps.

## TABLEAU 3

| Heures Fractions | 48 | 96 | 120 | 144 | 168 |
|---|---|---|---|---|---|
| SVF 10 % | $64.10^4$ | $86.10^5$ | $11.10^6$ | $94.10^5$ | $63.10^5$ |
| LFLac 50 10 % + SVF 1 % | $40.10^4$ | $20.10^5$ | $26.10^5$ | $42.10^5$ | $18.10^5$ |
| SVF 1 % | $24.10^4$ | $92.10^5$ | $92.10^4$ | $76.10^4$ | $52.10^4$ |

Les résultats obtenus montrent que 10 % de LFLac 50 supplémenté de 1 % de SVF présentent une activité équivalente à 10 % de SVF.

Exemple 6 : Utilisation de fractions de lait pasteurisé avec un système de fibroblastes fixés synchronisés.

On étudie l'incorporation de thymidine tritiée en opérant comme décrit ci-dessus.

Les résultats obtenus montrent également que LPLac E (phase intermédiaire obtenue après ultracentrifugation, non fractionnée) est moins actif que la fraction du même lait ne contenant que les molécules de PM supérieur à 50 000 daltons.

Exemple 7 : Fractionnement de lait ou de lait thermisé

Le lait cru recueilli de cinq vaches, deux mois après le début de la lactation, à 4° C, est transporté immédiatement au laboratoire où une partie est traitée entre 4 et 20° C pour les opérations qui suivent (cette

7

partie sera appelée lait cru ci-après).

L'autre partie est portée à 55° C pendant 30 minutes (on la désignera par lait thermisé).

Le lait cru et le lait thermisé sont soumis à une ultracentrifugation à 37600 g (ultracentrifugeuse KONTRON T 2080, rotor TFA 20, 19000 T/m) 4° C 60 mn, et la phase intermédiaire limpide récupérée est soumise à une deuxième ultracentrifugation identique pour bien éliminer les lipides et les protéines insolubles.

La phase intermédiaire limpide est répartie en aliquotes de 5 ml et conservée jusqu'à utilisation.

Une partie de la phase intermédiaire limpide précédemment obtenue est soumise telle quelle à deux ultrafiltrations successives, d'abord sur des membranes Millipore YM 10 à seuil de coupure de 1000 daltons, ensuite sur YM 2 à seuil de coupure de 10.000 daltons, et conduit à un rétentat A ou AT, respectivement avec le lait cru et le lait thermisé, une autre partie est soumise à une température d'environ 55° C pendant environ 30 mn avant d'effectuer les opérations d'ultrafiltrations ci-dessus, ce qui conduit respectivement aux rétentats B et BT.

Les rétentats B et BT sont lavés avec deux volumes d'eau apyrogène puis soumis à une ultrafiltration à seuil de coupure de 10.000. On obtient respectivement des rétentats C et CT que l'on ramène au volume initial.

**Exemple 8** : Utilisation comparée des échantillons de fractions de lait cru et de fractions de lait thermisé dans des tests biologiques sur des cellules d'hybridomes secrétant une immunoglobuline Ig G1.

A - Incorporation de thymidine $^3H$

Dans une microplaque de 96 puits, on répartit $5x10^4$ cellules par puit dans 200 µl de milieu RPMI 1640 complémenté par : L-glutamine 2mM, pyruvate de Na 1,2 mM, glucose 0,3%, βmercaptoéthanol 0,5 mM, pénicilline G 0,1µg/ml et streptomycine 0,05 µg/ml (concentrations finales).

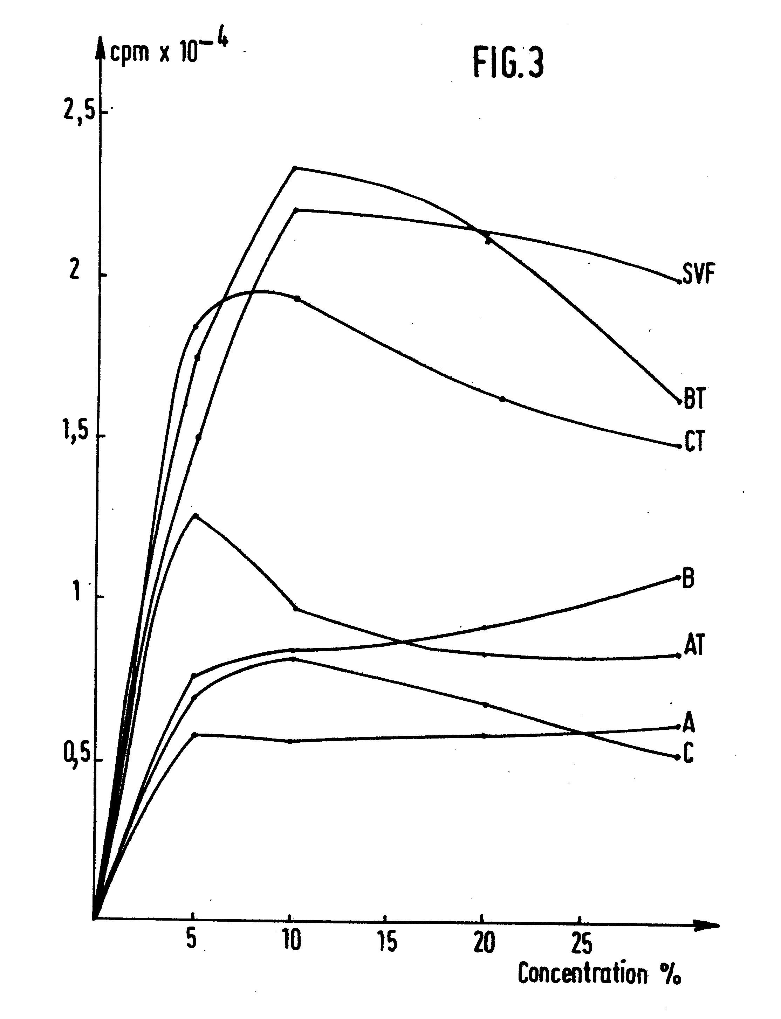

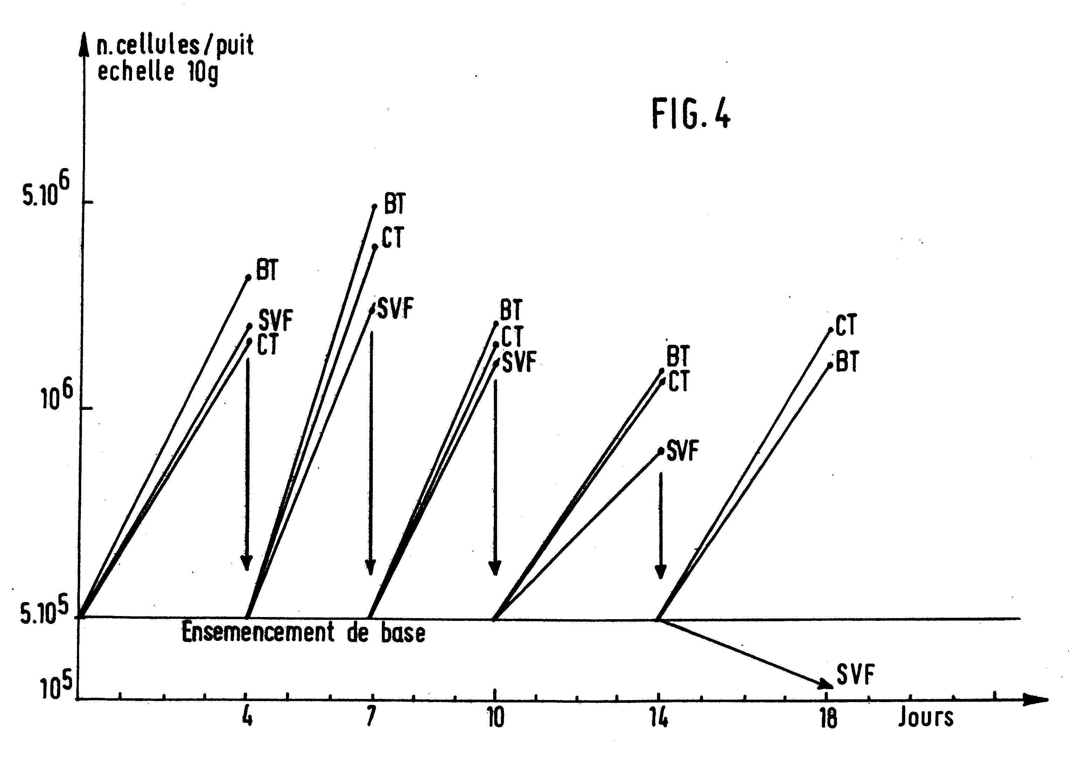

On ajoute les produits à tester aux concentrations finales voulues selon les expériences (voir les figures 3 et 4) puis on incube 24 heures à 37° C dans une étuve à $CO_2$ (5%).

On ajoute alors 50 µl/puit d'une solution de thymidine tritiée (AMERSHAM Act. Spécifique 1 mCi/ml) à la concentration finale de 1 µCi/puit. On laisse 18 heures à l'étuve avant de récupérer les cellules pour les laver dans un appareil automatique et mesurer la radioactivité.

La figure 3 donne les résultats obtenus avec les fractions A, B, C, AT, BT et CT, par rapport au sérum de veau foetal (SVF BOEHRINGER, lot 69337801).

Il en résulte que les fractions de lait thermisé donnent des incorporations plus importantes que les fractions correspondantes non thermisées en particulier entre 5 et 10% de concentration. De plus, la fraction BT donne des résultats comparables au SVF si ce n'est supérieurs, aux concentrations 5 et 10%.

B - Utilisation de fractions de lait thermisé selon l'invention, pour la culture de cellules d'hybridomes à long terme.

Les cellules sont cultivées en boîte de 75ml (FALCON) à fond plat contenant 7ml de milieu RPMI 1640 complémenté par : L-glutamine, pyruvate de Na, glucose, βmercaptoethanol, pénicilline G et streptomycine comme décrit ci-dessus.

On ensemence à l'origine chaque boite par $5x10^5$ cellules/ 7 ml et on mesure chaque jour la concentration cellulaire par comptage en cellule de THOMA. Tous les jours, les cellules sont reprises, diluées pour obtenir une concentration de $5x10^5$ cellules/ 7 ml et repiquées dans de nouvelles boites.

A chaque repiquage, on fera varier le taux respectif de SVF et des fractions de lait (les fractions BT et CT plus actives dans le test précédent, ont été retenues), c'est ainsi que :

| Jours | 1-2-3 | 4-5-6 | 7-8-9 | 10-11-12-13 | 14-15-16-17-18 |
|---|---|---|---|---|---|
| **Milieu contenant :** | | | | | |
| SVF | 7,5% | 5% | 2,5% | 1% | 0,5% |
| Fractions de lait | 2,5% | 5% | 7,5% | 9% | 9,5% |
| témoin SVF seul | 7,5% | 5% | 2,5% | 1% | 0,5% |

La figure 4 donne la concentration cellulaire dans la boite de culture chaque jour et l'on en déduit que au bout du 5ème passage (18ème jour de culture) les cellules d'hybridomes cultivées en présence de 0,5% de SVF + 9,5% de BT ou de CT, ont gardé leur potentiel de reproduction. A cette période, en présence de seulement 0,50% de SVF seul, les cellules périclitent. On montre que les cellules ont conservé également leur potentiel de secrétion de l'Ig G1.

Exemple 9 : Utilisation de fractions de lait en lieu et place de SVF, pour la conservation par congélation de cellules d'hybridome.

La souche cellulaire est une souche d'hybridome secrétant une immunoglobuline Ig G1 maintenue en culture dans le milieu décrit précédemment et contenant 0,5% SVF + 9,5% BT. On pratique la congélation comme à l'habitude mais on remplace le SVF par un mélange 0,5% SVF + 40% BT en volume, de telle sorte que le milieu de congélation est composé finalement de : SVF 0,5% + BT 40% + DMSO 10%.

Les cellules congelées à -80° C pendant 45 jours ont été décongelées comme habituellement et reprises dans le milieu de culture de départ contenant 0,5% SVF + 9,5% BT.

On a compté les cellules à 2 jours, 5 jours, 8 jours et 12 jours en ramenant à chaque comptage, la concentration à 5x10$^5$ cellules/ 7 ml.

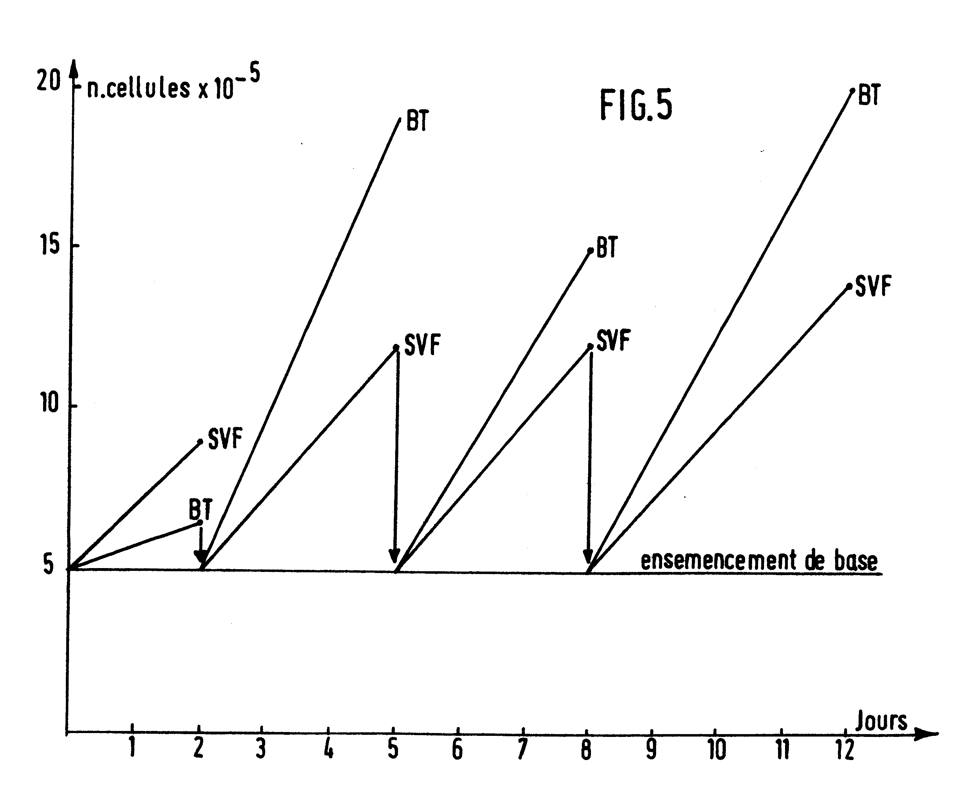

Les résultats obtenus rapportés sur la figure 5, montrent qu'à l'exception du temps 2 jours, les cellules congelées en milieu riche en BT, ont gardé, dans ce milieu, un fort potentiel de division. Ce potentiel est d'ailleurs plus important dans ce milieu que dans du SVF 10%.

**Revendications**

1. Fractions de lait, caractérisées en ce qu'elles sont formées de constituants ayant un PM d'au moins environ 27 000 et qu'elles sont essentiellement dépourvues de lipides insolubles du type de ceux éliminables par ultracentrifugation.

2. Fractions selon la revendication 1, caractérisées en ce qu'elles sont formées de constituants de PM supérieur à 50 000 environ.

3. Fractions selon la revendication 1, caractérisées en ce qu'elles sont formées de constituants de PM de 27 000 à 50 000 environ.

4. Fractions selon l'une quelconque des revendications 1 à 3, caractérisées en ce qu'elles sont soit entières, renfermant en mélange les constituants dont les PM sont supérieurs à 50 000, ou bien s'étagent de 27 000 à 50 000, soit fractionnées dans cet intervalle de PM.

5. Fractions de lait selon l'une quelconque des revendications 1 à 4, caractérisées en ce qu'il s'agit de fractions de lait frais, de lait pasteurisé, sous forme liquide concentrée ou lyophilisée.

6. Fractions de lait, caractérisées en ce qu'elles sont de type de celles obtenues par ultracentrifugation à une température inférieure à l'ambiante, en particulier inférieure à 10°C, notamment de l'ordre de 4°C, pendant une durée de l'ordre de 60 minutes, à 27 000 tours environ par minute, récupération de la phase intermédiaire et ultrafiltration de cette phase intermédiaire sur une membrane ayant un seuil de coupure approprié dans l'intervalle de 50 000 à 27 000 daltons.

7. Fractions de lait thermisé ayant subi un traitement de préchauffage à une température inférieure à 60° C environ, pendant au moins 5 à 10 minutes, essentiellement dépourvues de lipides insolubles du type de ceux éliminables par ultracentrifugation et formées de constituants ayant un PM d'au moins environ 10.000.

8. Fractions de lait selon la revendication 7, caractérisées en ce qu'elles sont du type de celles obtenues par ultracentrifugation du lait thermisé à 50.000g au plus, de préférence entre 30 et 40.000 g, suivie d'au moins une ultracentrifugation dans des conditions analogues de la phase intermédiaire limpide recueillie, puis d'au moins une étape d'ultrafiltration sur membranes afin d'éliminer les molécules de PM inférieur à environ 10.000.

9. Procédé de préparation de fractions selon l'une quelconque des revendications 1 à 8, caractérisé en ce qu'on soumet une phase limpide de lait, renfermant les protéines solubles du lait, telle qu'obtenue par ultracentrifugation du lait et séparation à partir des différentes phases obtenues, à une ultrafiltration avec une membrane à seuil de coupure de 50 000 à 27 000 daltons environ, et dans le cas de lait thermisé de 10.000 daltons environ.

10. Procédé selon la revendication 9, caractérisé en ce qu'on sépare tout d'abord à l'aide d'une membrane à seuil de coupure de 50 000 daltons la fraction de lait formée de constituants de PM supérieur à 50 000, puis on soumet la fraction restante à une ultrafiltration avec une membrane ayant un seuil de coupure approprié pour l'obtention d'une fraction formée de constituants présentant des PM dans l'intervalle de PM désiré jusqu'à 50 000 daltons.

11. Procédé selon la revendication 9 ou 10, caractérisé en ce que la fraction obtenue après ultrafiltration est concentrée, préfiltrée puis stérilisée.

12. Procédé selon l'une quelconque des revendications 9 à 11, caractérisé en ce que la phase de lait limpide mise en oeuvre dans l'étape d'ultrafiltration est obtenue avantageusement en soumettant le lait à au moins une opération d'ultracentrifugation, l'ultracentrifugation étant réalisée plus spécialement à 27 000 t/mn environ durant 60 mn environ, à une température inférieure à l'ambiante, avantageusement inférieure à 10°C, notamment de l'ordre de 4°C.

13. Milieux de cultures cellulaires caractérisés en ce qu'ils comprennent une fraction de lait selon l'une

9

quelconque des revendications 1 à 8.

14. Milieux de cultures selon la revendication 13, caractérisés en ce qu'ils renferment de 6 à 10 % environ des fractions de lait.

15. Milieux de cultures selon l'une quelconque des revendications 13 ou 14, caractérisés en ce qu'ils renferment en outre 1 à 10 % de sérum de veau foetal (SVF), de préférence de 3 à 5%.

16. Application des milieux de cultures selon l'une quelconque des revendications 13 à 15 à la culture de cellules d'eucaryotes.

17. Cellules d'eucaryotes sous forme congelée dans un milieu selon l'une quelconque des revendications 13 à 15, ces cellules pouvant être conservées et remises en culture dans ce milieu.

FIG.1

FIG.2

0219372

FIG.3

FIG. 4

n.cellules/puit
echelle 10g

$5.10^6$

$10^6$

$5.10^5$

$10^5$

Ensemencement de base

BT
SVF
CT

BT
CT
SVF

BT
CT
SVF

BT
CT
SVF

CT
BT

SVF

4    7    10    14    18    Jours

0219372

FIG.5

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | GB-A- 983 489 (USTAV SER A OCKOVACICH LATEK PRAHA) * Revendications 1,3; page 3, tableau 3 * | 1,13 | C 12 N 5/00 A 23 C 9/142 |
| A | FR-A-2 292 435 (INSTITUT NATIONAL DE LA RECHERCHE AGRONOMIQUE) * Revendications 1,2,5; exemple 1 * | 1,5 | |
| A | FR-A-2 557 886 (INSTITUT NATIONAL DE LA SANTE ET DE LA RECHERCHE MEDICALE) * Revendications 1,6,10; page 5, lignes 7-9 * | 1,13 | |
| A,D | US-A-4 440 860 (M. KLAGSBRUN) * Revendications 1,3; colonne 6, tableau * | 1,5,13 | |

DOMAINES TECHNIQUES RECHERCHES (Int. Cl.4)

C 12 N
A 23 C
A 23 J

Le présent rapport de recherche a été établi pour toutes les revendications

| Lieu de la recherche | Date d'achèvement de la recherche | Examinateur |
|---|---|---|
| LA HAYE | 15-12-1986 | PEETERS J.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur. mais publié à la date de dépôt ou après cette date
D : cité dans la demande
L : cité pour d'autres raisons

& : membre de la même famille. document correspondant

OEB Form 1503 03 82